# EUROPEAN PATENT SPECIFICATION

(11) **EP 2 750 582 B1**
(45) Date of publication and mention of the grant of the patent: **21.08.2019**
(21) Application number: 12753650.6
(22) Date of filing: 24.08.2012
(51) Int. Cl.: A61B 1/00, A61B 1/06

(54) **DISPOSABLE SHEATH WITH LIGHTING**
EINWEGHÜLLE MIT BELEUCHTUNG
GAINE JETABLE AVEC ÉCLAIRAGE

(30) Priority: 31.08.2011 US 201161529654 P
(43) Date of publication of application: 09.07.2014
(73) Proprietor: Boston Scientific Scimed, Inc., Maple Grove, MN 55311-1566 (US)
(72) Inventor: WEITZNER, Barry, Acton, MA 01720 (US); SMITH, Paul, J., Smithfield, RI 02917 (US); DEVRIES, Robert, B., Northborough, MA 01532 (US)
(74) Representative: Vossius & Partner Patentanwälte Rechtsanwälte mbB
(86) International application number: PCT/US2012/052194
(87) International publication number: WO 2013/032880

(56) References cited:
- WO-A1-2010/111461
- US-A1- 2004 111 009
- US-A1- 2007 270 646
- US-A1- 2011 201 889

## Description

### Field of the Invention

Embodiments of the present invention relate to a disposable sheath for a device. In particular, exemplary embodiments of the present invention relate to disposable guide sheaths that allow devices to be used within the body of a patient without direct tissue contact.

### Background of the Invention

Medical devices such as endoscopes and other similar devices may be used for diagnostic and therapeutic applications within the body of a patient. While some of these medical devices ("devices") are disposable devices that are discarded after a single use, others may be reusable. The reusable devices may have to be sterilized between uses to prevent cross contamination between patients. To reduce issues associated with sterilization and contamination, some of these devices may be covered with sterile disposable sheaths during use. Such sheaths may be elongated, tubular components having a lumen for the insertion of the device. During use, the sheath may isolate the device from body tissue. After use, the disposable sheath is removed from the device and discarded. Since the devices themselves are not in contact with body tissue, the likelihood of contamination is minimized. Examples of a sheath for a medical device are disclosed in US 2004/0092794 A1, US 2007/0270646 A1 and WO2010/111461 A1.

In some cases, it may be desirable to direct multiple devices into the body to assist in a medical application. Some of these devices may be disposable while others may be fully or partially reusable. Introducing these multiple devices into the body individually may be inefficient and may also increase patient discomfort and trauma. To minimize patient discomfort and improve the efficacy of the medical procedure, it may be advantageous to direct these multiple devices into the body through the same disposable sheath. In these cases, it may be desirable to isolate reusable devices from disposable devices to minimize contamination. Embodiments of the current disclosure are directed to disposable sheaths that enable multiple devices to be used alongside each other within the body.

### SUMMARY OF THE INVENTION

An embodiment of the invention discloses a sheath for a medical device including an elongate body extending from a proximal end to a distal face and a first hollow lumen and a second hollow lumen extending from a first end proximate the proximal end to a second end proximate the distal face. The sheath may also include at least one lighting device positioned in the sheath proximate the distal face, and a transparent window positioned at the second end of the first lumen to seal the first lumen at the second end. The transparent window may be configured to transmit light therethrough.

Various embodiments of the invention may include one or more of the following aspects: the at least one lighting device may include a plurality of lighting devices spaced apart from each other; the plurality of lighting devices may be symmetrically positioned with respect to a longitudinal axis of the sheath; the at least one lighting device includes a plurality of lighting devices positioned as a ring along a periphery of the sheath; the sheath may further include a second transparent window positioned at the second end of the second lumen to seal the second lumen at the second end, the transparent window being configured to transmit light therethrough; transparent window at the second end of the first lumen and the second transparent window at the second end of the second lumen may be part of a same transparent window; the sheath may include a first neck region that extends from the distal face to the second end of the first lumen, at least a portion of the first lumen may be positioned in the first neck region; the sheath may further include a second neck region that extends from the distal face to the second end of the second lumen, at least a portion of the second lumen may be positioned in the neck region; and the at least one lighting device may be positioned proximate a periphery of the second end of the first lumen; the sheath may further include a third lumen extending from the proximal end to the distal face, the third lumen may be open at the distal face.

An embodiment of the invention may disclose a medical device, including a flexible sheath extending from a proximal end to a distal face and a first hollow lumen extending through the flexible sheath from the proximal end to the distal face. The sheath may also include at least one lighting device positioned at the distal face, and a neck region that extends the first lumen from the distal face of the sheath to a second end that is distal to the distal face of the sheath. The first lumen may be closed at the second end by a transparent window. The sheath may further include a second lumen extending from the proximal end to the distal face of the sheath.

Various embodiments of the invention may include one or more of the following aspects: the at least one lighting device may include a plurality of lighting devices spaced apart from each other; the at least one lighting device may include a plurality of lighting devices arranged as a ring around a longitudinal axis of the sheath; the sheath may further include a third hollow lumen extending through the flexible sheath from the proximal end to a third end proximate the distal face; and the sheath may further include a second transparent window positioned at the third end of the third lumen to seal the third lumen at the third end, the transparent window may be configured to transmit light therethrough.

Another embodiment may disclose a method of using a medical device. The method may include inserting a distal end of a sheath into the body of a patient. The sheath may include an elongate body extending from a proximal end to the distal end and at least one lighting device positioned proximate the distal end. The sheath may also include a first hollow lumen and a second hollow lumen extending through the sheath from the proximal end to the distal end, the first lumen may be sealed at the distal end by a transparent window. The method may also include inserting a first medical device having an optical device into the first lumen of the sheath either before, during, or after the sheath is inserted into the body, and positioning the optical device of the first medical device proximate the transparent window. The method may further include activating the at least one lighting device to illuminate a region distal to the distal end.

Various embodiments may include one or more of the following aspects: the method may further include inserting a second medical device through the second lumen of the sheath to extend an end effector of the second medical device out of the distal end of the sheath; activating the optical device of the first medical device to record an image of a region distal to the distal end of the sheath through the transparent window; and adjusting an illumination level of the at least one lighting device based on the recorded image.

### BRIEF DESCRIPTION OF THE DRAWINGS

The accompanying drawings, which are incorporated in and constitute a part of this specification, illustrate embodiments of the invention and together with the description, serve to explain the principles of the invention.
FIG. 1 is a schematic view of an exemplary embodiment of a sheath of the current disclosure.
FIG. 2A is a schematic illustration of the distal end of an exemplary embodiment of a sheath of the current disclosure.
FIG. 2B is a schematic illustration of the distal end of another exemplary embodiment of a sheath of the current disclosure.
FIG. 2C is a schematic illustration of the distal end of another exemplary embodiment of a sheath of the current disclosure.
FIG. 2D is a schematic illustration of the distal end of another exemplary embodiment of a sheath of the current disclosure.
FIG. 2E is a schematic illustration of the distal end of another exemplary embodiment of a sheath of the current disclosure.
FIG. 3A is a schematic illustration of the distal end of an exemplary embodiment of a sheath with an expandable section.
FIG. 3B is a schematic illustration of the sheath of the sheath of FIG. 3A with the expandable section activated.
FIG. 4 is a schematic illustration of the distal end of another exemplary embodiment of a sheath of the current disclosure.
FIG. 5 is a schematic illustration of the distal end of another exemplary embodiment of a sheath of the current disclosure
FIG. 6A is a schematic illustration of the proximal end of an exemplary embodiment of a sheath of the current disclosure.
FIG. 6B is a schematic illustration of the proximal end of another exemplary embodiment of a sheath of the current disclosure.
FIG. 7A is a schematic view of an exemplary embodiment of a sheath with a device having disposable and reusable portions.
FIG. 7B is a schematic view of an exemplary embodiment of the interface between the disposable and reusable portions of the device of FIG. 2A.
FIG. 8 is a schematic view of an exemplary reusable device that may be used with a sheath of the current disclosure.
FIG. 9 is a schematic illustration of the reusable device of FIG. 8 used with an embodiment of a sheath of the current disclosure.
FIG. 10 is a flow chart illustrating an exemplary medical procedure using a sheath of the current disclosure.

It should be noted that the dimensions of the assemblies shown in the figures may be distorted for clarity of the illustration, and different proportions of the different dimensions are also possible, and like reference numbers in different figures represent similar elements.

### DESCRIPTION OF THE EMBODIMENTS

Reference will now be made to exemplary embodiments of the invention, examples of which are illustrated in the accompanying drawings. Wherever possible, the same reference numbers will be used throughout the drawings to refer to the same or like parts. The terms "proximal" and "distal" are used herein to refer to the relative positions of a device. As used herein, "proximal" refers to a position relatively closer to the exterior of the body or closer to a user (surgeon, physician, etc.) using the device. In contrast, "distal" refers to a position relatively further away from the user (surgeon, physician, etc.) using the device or closer to the interior of the body.

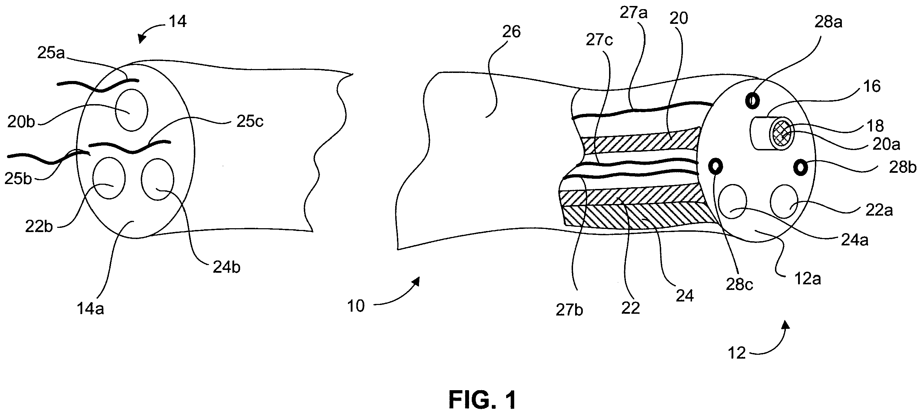

FIG. 1 depicts an exemplary guide or sheath 10 of the current disclosure. Sheath 10 may include a flexible elongate body 26 that extends between a proximal end 14 and a distal end 12. During application, the distal end 12 may be positioned at a work site within the body of a patient while the proximal end 14 may extend outside the body. The distal end 12 of the sheath 10 may be inserted into the body of the patient and pushed into the body (such as, for example, through a body tract) to position the distal end 12 at a desired work site within the body. The sheath 10 may be inserted into the body transluminally, intraluminally, percutaneously or by any other means. For example, the distal end 12 of sheath 10 may be inserted into the body of a patient through the mouth and pushed into the body through the esophagus to position the distal end 12 proximate an ulcer on the stomach wall, while the proximal end 14 is positioned outside the body. Elongate body 26 may be flexible so as to enable the sheath 10 to bend and pass through tortuous body tracts as the distal end 12 advances into the body through the body tract. In some embodiments, an external surface of elongate body 26 may be made of, or coated with, a polymeric or a lubricious material (such as, for example, MDX coating, PTFE extrusions, etc.) to enable the sheath 10 to pass through body cavities with ease. It is also contemplated that, in some embodiments, an external surface of the elongate body 26 is coated with (or provided with) medicines (such as, antibiotics, etc.) to assist in healing. In some embodiments, elongate body 26 may have surface modifications (bumps, ribs,. etc.) to ease insertion and extraction into the body.

One or more lumens (such as, for example, first lumen 20, second lumen 22, and third lumen 24) may extend through the elongate body 26 from the proximal end 14 to the distal end 12. In general, any number of lumens may extend through the elongate body 26. While one or more of these lumens may be sealed at the distal end 12 to prevent the entry of biological fluids thereinto, the other lumens may be open. For instance, first lumen 20 may be sealed at the distal end 12 as illustrated in FIG. 1. An optically transparent window (such, as for example, a viewing lens 18) may be provided at the distal end 12 of first lumen 20 to allow light to pass therethrough, while isolating the interior of first lumen 20 from body tissue and fluids. Any type of material that allows at least a portion of light to pass therethrough may be used as viewing lens 18. In some embodiments, viewing lens 18 may incorporate features such as, magnification, filtering, polarizing, etc. During application, a device (such as, for example, an endoscope, a camera, a light wand, etc.) may be directed into the body through first lumen 20. This device may be inserted into the first lumen 20 through an opening 20b at the proximal end 14 of the sheath 10. The device may then be pushed into the body through the first lumen 20 to position its distal end proximal the viewing lens 18. In some embodiments, as illustrated in FIG. 1, at the distal end 12, the first lumen 20 may include a protruding section or a neck region 16 that extends past a distal face 12a of the sheath 10. In these embodiments, the distal-most end 20a of the first lumen 20 may be longitudinally displaced from the distal face 12a, and viewing lens 18 may be provided at the distal-most end 20a. In some embodiments, the device that is inserted through the first lumen 20 may be a reusable device. Although, in general, any type of device (reusable, disposable, etc.) may be directed through the sealed first lumen 20, in this description, the device directed through the sealed first lumen 20 will be referred to as a reusable device.

As opposed to the first lumen 20 which is sealed at the distal end 12 and open at the proximal end 14, the second lumen 22 and the third lumen 24 may be open at both the proximal end 14 and the distal end 12. One or more devices may be inserted into the second and the third lumens 22, 24 through openings 22b, 24b at the proximal end 14. These devices may then be pushed into the sheath 10 to extend the distal ends of the devices into the body through openings 22a, 24a at the distal end 12 of sheath 10. The second lumen 22 and the third lumen 24 may provide access for devices that may aid in performing any desired medical procedure within the body. In some embodiments, as illustrated in FIG. 1, the openings 22a and 24a of the second and the third lumens 22, 24 may be positioned on distal face 12a. In other embodiments, however, the distal ends of the second and third lumens 22, 24 may also include protruding sections (similar to neck region 16 of first lumen 20) that extend the openings 22a and 24a past the distal face 12a. Although the distal ends of the second and third lumens 22, 24 are described as being open, in some embodiments, the openings 22a, 24a at the distal ends of these lumens may include seals or other mechanisms that may prevent or decrease the entry of biological fluids into the lumens while allowing a device to pass therethrough. These seals may be active or passive seals. In some embodiments, a distal end of a device inserted through the second and third lumens 22, 24 may penetrate or pierce through a seal to extend out of the distal end of the lumen. The seal may snugly fit around a portion of the device that extends out of the lumen to prevent or minimize the entry of biological fluids into the lumen.

Sheath 10 may be made of any material (metal, plastic, polymeric, etc.) and have any stiffness (of flexibility) to suit an application. In some embodiments, the sheath 10 may be a relatively floppy component, while in other embodiments the sheath 10 may be relatively rigid. It is also contemplated that, in some embodiments, selected portions of the sheath 10 may be relatively rigid while other portions may be relatively flexible. The increased rigidity at the selected portions may be provided by incorporating different materials into the sheath 10 or by incorporating features (stiffening ribs, thicker regions, etc.) that impart rigidity to selected regions of the sheath 10. In some embodiments (such as, for example, when sheath 10 is relatively floppy), a device may be inserted into a lumen of the sheath 10 before the sheath 10 (along with the inserted device) is inserted into the body of the patient. In such an embodiment, the stiffness provided by the inserted device may guide the sheath 10 into the body. After the sheath 10 is appropriately positioned within the body, other devices may be inserted through other lumens of the sheath 10. For example, in one exemplary embodiment, a reusable device may be inserted into the sealed first lumen 20 of a relatively floppy sheath 10 before the sheath 10 is inserted into a body cavity. Once the distal end 12 of the sheath 10 is appropriately positioned in the body, the disposable devices may be inserted through the second and third lumens 22, 24. It is also contemplated that the disposable devices are also inserted into the sheath 10 before the sheath 10 is inserted into the body cavity.

In some embodiments, sheath 10 may be inserted into the body and positioned at a desired location in the body before the disposable and reusable devices are inserted into the lumens of the sheath 10. In such an embodiment, the inserted sheath 10 acts as a guide to direct the devices into the body. It is also contemplated that, in some embodiments, one or more devices or guides may be inserted into the body, and the sheath 10 may be slid over the inserted device from the proximal end of the device. For example, in some exemplary applications, a device may first be inserted into the body (individually inserted or inserted through a guide tube, etc.). The proximal end of the inserted device (located outside the body) may be inserted into the distal end of the sheath (through an open lumen), and the sheath 10 pushed into the body until the distal end 12 is positioned at a desired location within the body. In such an embodiment, the inserted device may guide the sheath 10 to the desired location in the body. Once positioned at the desired location, the reusable device may then be inserted through the sealed first lumen 20.

The distal end 12 of sheath 10 may also include one or more lighting devices, such as, for example, lighting devices 28a, 28b, and 28c. These lighting devices may include any device that is adapted to illuminate the work site within the patient's body, and may include permanent, removable, or replaceable devices. In some embodiments, sheath 10 may be reflective or may have reflective regions that aid in distribution of light *in vivo.* It is also contemplated that sheath 10 may include markers (lines, dots, etc.) that enhance the visibility of the sheath in the body under illumination. The lighting devices 28a, 28b, and 28c may include LEDs, fiber optic cables, bulbs, or any other device that emits light. In some embodiments, each of the lighting devices 28a, 28b, and 28c may be configured to provide substantially the same amount of light. While in other embodiments, the light emitted through each of the lighting devices may be separately controlled. Cables, such as, for example, cables 27a, 27b, and 27c may extend through sheath 10 to couple the lighting device 28a, 28b, and 28c to proximal end 14 of sheath 10. These cables 27a, 27b, and 27c may direct power and signals to and from the lighting devices 28a, 28b, and 28c. At the proximal end 14, these cables 27a, 27b, and 27c may extend out of sheath 10 and may be configured to be connected to a control device (not shown) that provides power to, and controls, the lighting devices 28a, 28b, and 28c. In some embodiments, a fiber optic element may extend from the proximal end 14 to the distal end of sheath 10. In these embodiments, the fiber optic element may extend to, or out of, the distal end 12 and may serve as a lighting device.

Although FIG. 1 illustrates a separate cable for each lighting device 28a, 28b, and 28c as emerging from the proximal end 14 of sheath 10, this is not a requirement. In some embodiments, the number of cables may be more than or less than the number of lighting devices. For example, in some embodiments, a single cable may extend out of, and couple, multiple lighting devices (such as, the three lighting devices 28a, 28b, and 28c of FIG. 1) to the control device at the proximal end 14. In some embodiments (such as, for example with fiber optic lighting devices) a separate cable may be eliminated. The control device may provide power and signals to the lighting devices 28a, 28b, and 28c to provide sufficient illumination at the work site. In some embodiments, the control device may determine a desired illumination pattern for the work site based on received input (such as, for example, based on the illumination level in an acquired image of the work site), and control the individual lighting devices 28a, 28b, and 28c to provide the desired illumination pattern. In such embodiments, the control device may control the individual lighting devices 28a, 28b, and 28c separately to provide a different level of illumination from some or all of the lighting devices 28a, 28b, and 28c. For example, due to different light reflecting characteristics of different regions within the work site (such as, fat, tissue, organs, etc.), an image of the work site may indicate that, for increased clarity, some regions of the work site should be lighted differently from other regions. For example, the acquired image may indicate that some regions should be lighted more while other regions should be lighted less, or some regions to be lighted with light of a different color as compared to another region. Based on this input, the control device may control the individual lighting devices 28a, 28b, and 28c to selectively increase and/or decrease the illumination level of different lighting devices 28a, 28b, or 28c. The illumination level may be varied by any method. For instance, in some embodiments, the control device may vary a characteristic of the light (such as, for example, frequency, wavelength, amplitude, phase shifting, color, etc.) emitted by different lighting devices 28a, 28b, 28c, to vary the illumination level of the different devices. In some embodiments, the control device may control the lighting devices 28a, 28b, 28c, to create effects such as strobing.

Although FIG. 1 illustrates the proximal ends 25a, 25b, and 25c of cables 27a, 27b, and 27c as emerging from proximal end 14 of sheath 10, this is not a requirement. In some embodiments, some or all these cables may terminate at a plug or a connection feature provided at the proximal end 14. In such embodiments, a mating plug or connection feature may couple with the proximal end of the cable to electrically couple the lighting devices 28a, 28b, and 28c to the control device.

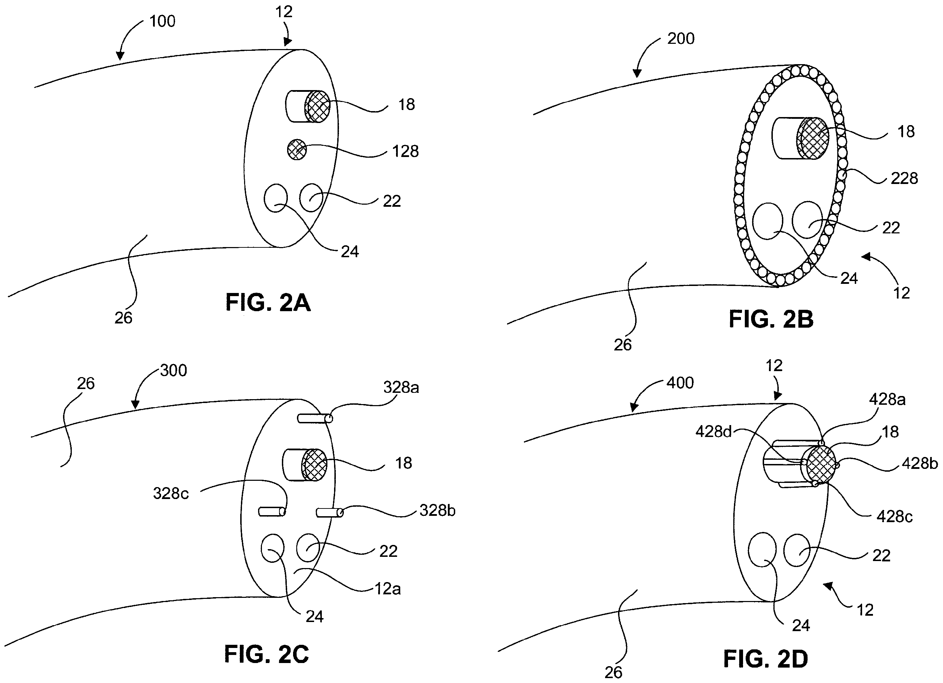

Any number of lighting devices may be provided at the distal end 12 of sheath 10, and these lighting devices may be positioned in any pattern at the distal end 12. FIGS. 2A-2E illustrate exemplary embodiments of sheaths illustrating several exemplary lighting devices at the distal end 12. In the description of the FIGS. 2A-2E below, reference will also be made to FIG. 1. Like reference numbers in FIGS 2A-2E and FIG. 1 represent similar elements, and for the sake of brevity, may not be described again. In some embodiments, as illustrated in FIG. 2A, a single lighting device 128 may be provided at the distal end 12 of sheath 100. The single lighting device 128 may be of any shape, size, and configuration (round, square, polygon, donut-shaped, etc.). This single lighting device 128 may be positioned at any location at the distal end 12. As illustrated in FIG. 2B, in some embodiments, a ring light 228 may be provided at the distal end 12 of a sheath 200. Such a ring light 228 may include several LEDs, fiber optic cables, or other illumination devices arranged in a pattern at the distal end 12. Although a ring light 228 that extends completely around a perimeter of sheath 200 is disclosed, this is not a requirement. For instance, in some embodiments, these lighting devices may extend only partially around, or in selected segments of, sheath 200.

Although FIGS. 2A and 2B illustrate lighting devices positioned on distal face 12a, this is not a requirement. In some embodiments, as illustrated in FIG. 2C, a sheath 300 may include one or more lighting devices (such as, for example, lighting devices 328a, 328b, and 328c) that protrude past the distal face 12a of sheath 300. In some such embodiments, the relative positions of the different lighting devices 328a, 328b, and 328c with respect to each other and/or with respect to distal face 12a may be changed by controlling the protruding region of each lighting device. In such embodiments, an actuating device at the proximal end of sheath 300 may be configured to change the position or orientation of one or more of the different lighting devices 328a, 328b, and 328c. In some embodiments, the actuating device at the proximal end of sheath 300 may control one or more of the lighting devices 328a, 328b, 328c, to move together or individually. For example, the actuating device may control a lighting device to extend or retract axially, move radially, or any combinations thereof. In some embodiments, as illustrated in sheath 400 of FIG. 2D, multiple lighting devices may be positioned around the sealed first lumen 20. Although four lighting devices 428a, 428b, 428c, and 428d are shown symmetrically positioned around first lumen 20 of FIG. 2D, this is only exemplary. For instance, a partial or a full ring light (such as ring light 228 of FIG. 2B) may be positioned around first lumen 20.

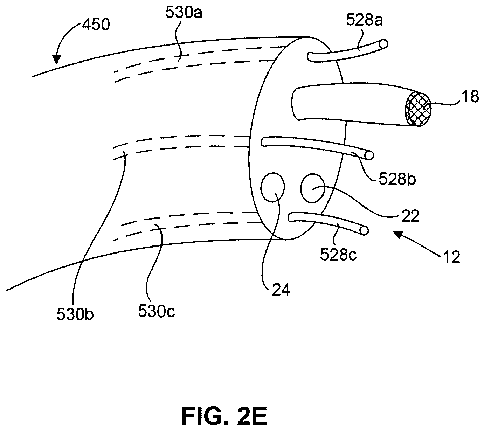

In some embodiments, as illustrated in sheath 450 of FIG. 2E, one or more of the lighting devices 528a, 528b, 528c may extend from the proximal end 14 to the distal end 12 of sheath 450 through their own separate channels 530a, 530b, 530c. These lighting devices may be configured to extend out of, and retract into, their respective channels. In some embodiments, these lighting devices may also be configured to move radially in the work site (that is, towards and away from a central axis of sheath 450). Some or all of these lighting devices 528a, 528b, 528c may be independently steered and controlled from the proximal end 14 of the sheath 450.

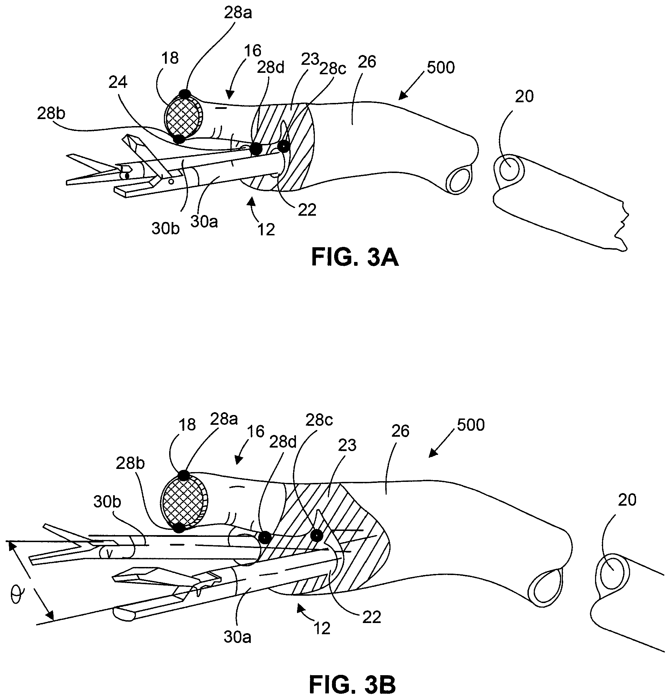

In some embodiments, as illustrated in sheath 500 of FIGS. 3A and 3B, a diverging element 23 may be included in the elongate body 26 to enable devices 30a and 30b in the second and third lumens 22, 24 to diverge from each other at the distal end 12. The diverging element 23 may be an active or a passive device. FIG. 3A illustrates a view of sheath 500 before diverging element 23 is activated, and FIG. 3B illustrates a view of the sheath 500 after the activation of diverging element 23. Activation of the diverging element 23 enables the ends of the devices 30a, 30b that extend out of the second and third lumens 22, 24 to diverge from each other by any angle θ. Activation of the diverging element 23 may also change the orientation of one or more of the illumination devices 28a, 28b, 28c, and 28d to change the illumination pattern of the work site and account for the divergence of the devices 30a and 30b. Although FIGS. 3A and 3B illustrate the devices 30a and 30b in the second and third lumens 22, 24 as diverging only from each other, in some embodiments, the diverging element 23 may also be configured to enable these devices 30a, 30b to diverge from the distal end of the reusable device in the first lumen 20 also. Although the diverging element 23 may be positioned at any location in sheath 500, in some embodiments, the diverging element 23 may be positioned proximate the distal end 12 of the sheath 500 to enable the distal ends of the devices delivered through sheath 500 to diverge from each other. The ability of the devices that extend through sheath 500 to diverge from each other at the distal end may further assist the user in performing a medical procedure within the body.

Any feature that enables the devices in the different lumens to diverge from each other may be used as diverging element 23. In some embodiments, the diverging element 23 may include a flexible section (such as a section made of a relatively more flexible material and/or having a smaller thickness) of elongate body 26 that can flex to enable the different devices to separate from each other. In other embodiments, the diverging element 23 may include sections of the elongate body 26 having a variable flexibility. For instance, a section of the elongate body 26 proximate diverging element 23 may include balloons or cavities that may be selectively filled with air (or another fluid) to vary the flexibility in that region. As the balloons or cavities are filled, they also may enlarge, pushing the lumens 22, 24 away from one another if, for example, the balloon or cavity is between the lumens 22, 24. The balloons or cavities may be connected to a source of fluid located at the proximal end of the sheath 500, through a lumen extending through the sheath 500. In other embodiments, diverging element 23 may include sections of elongate body 26 in which the flexibility may be selectively varied thermally, electrically, or by another method. It is also contemplated that, in some embodiments, devices such as, for example, actuators may serve as the diverging element 23. Diverging element 23 may involve shapes or geometries to promote divergence. In some embodiments, the diverging element 23 may create one or more ramps adjacent to a lumen to guide a device in the lumen in a particular direction. In some embodiments, the diverging element 23 may be bubble shaped, spherical or hemi-spherically shaped features to allow devices to glance off the diverging element 23 and be diverted relative to another device. In some embodiments, different diverging elements 23 may be associated with different lumens and may be positioned at different locations in sheath 500. The diverging element 23 may enable the distal ends of the devices to diverge from each other and further assist in a medical procedure.

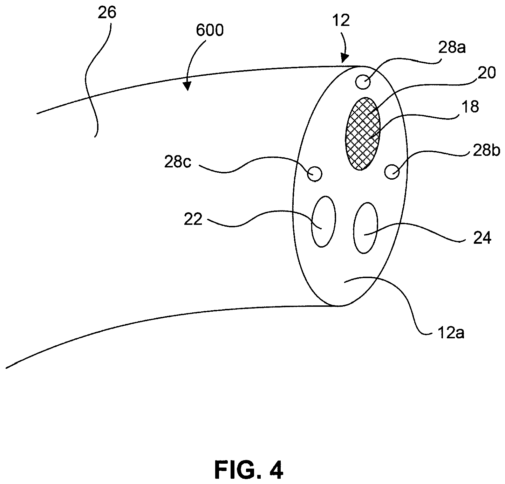

Although a protruding neck region 16 is illustrated in FIGS. 1, 2A-2D, 3A, and 3B, in some embodiments, as illustrated in sheath 600 of FIG. 4, the neck region 16 of the first lumen 20 may be eliminated. In such embodiments, the first lumen 20 may terminate at the distal face 12a, and the viewing lens 18 may be provided at the distal face 12a. Although one sealed lumen (first lumen 20) and two open lumens (second lumen 22 and third lumen 24) are illustrated in FIG 4, this is only exemplary. In general, any number of sealed and open lumens may be provided in sheath 600. Further, although these lumens are illustrated as being circular, in general, these lumens can have any cross-sectional shape. The cross-sectional shape may be fixed along the length or may vary along the length of elongate body 26. The change in cross-sectional shape along the length may be stepwise or continuous. In some embodiments, some or all lumens may be lined with a polymeric (or another) layer or coating (such as, for example, a lubricious coating such as MDX coatings, PTFE extrusions, etc.) to facilitate use. In some embodiments, some or all of these lumens may include surface modifications (bumps, ribs, etc.) to reduce friction. Further, the first, second, and third lumens 20, 22, 24 may be arranged in any pattern in sheath 600. In some embodiments, the first, second, and third lumens 20, 22, 24 may be arranged in a straight line (such as, for example, aligned along a vertical or a horizontal axis) or arranged in another pattern, such as, for example, positioned at the vertices of a triangle as illustrated in sheath 600 FIG. 4. It should be noted that the lumens extending through a sheath of the current disclosure may be arranged in any pattern, without limitation.

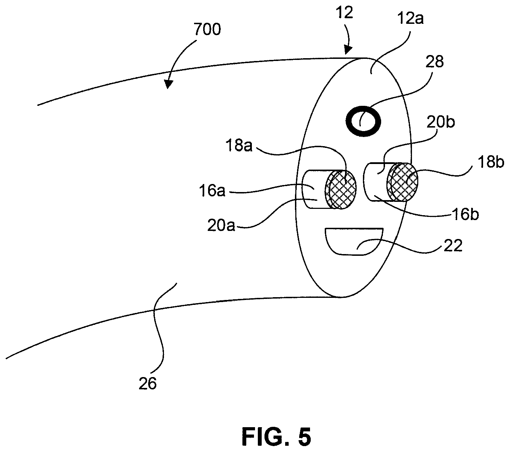

FIG. 5 illustrates another embodiment of sheath 700 having two first lumens (lumens 20a and 20b) that are sealed at the distal end 12, and one second lumen (lumen 22) that is open at the distal end 12. Both the first lumens 20a and 20b may include viewing lens 18a, 18b positioned at the distal-most end thereof. In some embodiments, the distal-most end of the first lumens 20a, 20b may lie on distal face 12a, while in other embodiments (as illustrated in FIG. 5), the distal ends of the first lumens 20a, 20b may include protruding neck regions 16a, 16b that extend the distal-most end of the first lumen 20a, 20b past the distal face 12a. One or more reusable devices (such as endoscopes, cameras etc.) may be introduced into the body through the multiple first lumens 20a, 20b. Providing multiple viewing devices through the two first lumens 20a, 20b may allow for binocular vision and help with depth perception. Although, a separate viewing lens (such as, viewing lens 18a and 18b) are shown to be provided for each first lumen 20a, 20b, this is only exemplary. It is also contemplated that a single viewing lens that extends across the two first lumens 20a, 20b may alternatively be provided at the distal end 12 of sheath 700.

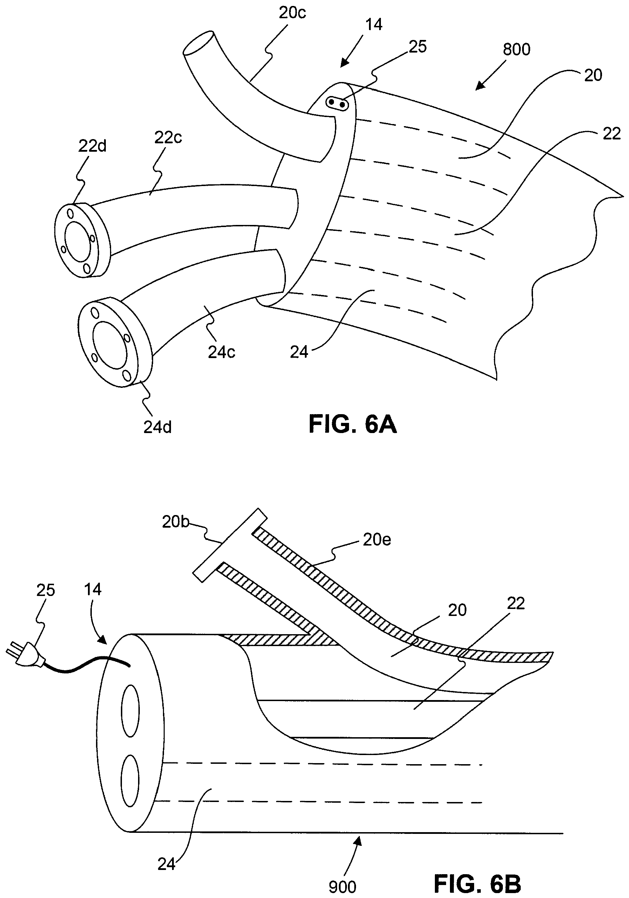

Although in FIG. 1, the proximal ends of the first, second, and third lumens 20, 22, 24 are illustrated as terminating (at openings 20b, 22b, and 24b, respectively) at proximal end or proximal face 14a, and the cables 25a, 25b, and 25c are illustrated as extending past the proximal face 14a, this is only exemplary. In some embodiments, as illustrated in sheath 800 of FIG. 6A, the cables 27a, 27b, and 27c (see, FIG. 1) that extend through the sheath 800 may terminate at a plug 25 at the proximal face 14a. A mating plug from a control device may couple to plug 25 to electrically couple the illumination devices at the distal end (not visible in FIG. 6A) of sheath 800 to the control device. One or more of the lumens that extend through sheath 800 may include projecting portions (such as, for example, 20c, 22c, and 24c) that project past the proximal face 14a. The projecting portions may be flexible or rigid and some or all of these projecting portions may include seals (such as, for example, seals 22d and 24d on projecting portions 22c and 24c respectively) or other fixtures. In some embodiments, these projecting portions of the lumens may mate with other equipment that may assist in the medical procedure. For example, in an exemplary medical procedure, second lumen 22 may be an irrigation lumen configured to facilitate fluid flow to a work site within the body, and the third lumen 24 may be an aspiration lumen configured to provide suction to the work site. In such an embodiment, the projecting portion 22c of the second lumen 22 may be attached to a fluid tank (or another source of fluid), and the projecting portion 24c of the third lumen 24 may be attached to a suction pump (or another device that provides suction). The seals 22d and 24d may assist in providing a fluid tight or an air tight connection between the fluid source/suction pump and the corresponding lumen. In some such embodiments, the distal ends of the second and third lumens 22, 24 may also include devices that may assist in its functioning. For example, a nozzle (or another similar device configured to alter fluid flow) may be attached to the distal end of second lumen 22 to assist in impinging the fluid at a desired location. Fluid may be directed to the work site through the second lumen 22 and suction may be provided through the third lumen 24 to remove the fluid (and/or biological material) from the work site. In this manner, a tissue sample may be extracted out of the body through the third lumen 24.

In some embodiments, such as, for example in sheath 900 of FIG. 6B, a port 20e positioned between the proximal and distal ends 14, 12 of the sheath 900 may provide access to the first lumen 20. This port 20e may extend at an angle from the surface of the elongate body 26. In some embodiments one or more of the cables that extend from the illumination devices at the distal end 12 to the proximal end 14 may join together and extend past the proximal end to terminate at a plug 25. Plug 25 may couple the illumination devices to a control device.

Any type of device known in the art may be provided through the second and the third lumens 22, 24. These devices may be configured to perform specific tasks to assist in the desired medical procedure. In some cases, an end effector that is configured to perform the task may be coupled to an elongate member that extends into the body through the second and the third lumens 22, 24. The elongate member may operatively couple the end effector to an actuation device at the proximal end 14 to operate the end effector. Since the distal ends of the second and third lumens 22, 24 are open, the devices directed into the body through these lumens may directly contact body tissue. Therefore, in some exemplary medical procedures using a disclosed sheath, these devices may be sterilized or may be disposed (along with the sheath) after use.

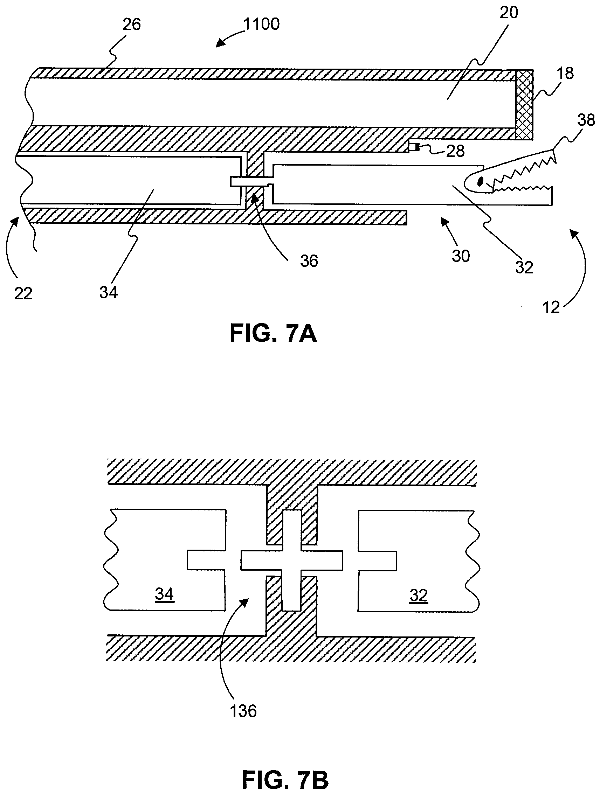

In some embodiments of a sheath, a device directed through the second or third lumen 22, 24 may be only partially disposable with a remainder of the device being reusable. In these embodiments, a portion of the device that contacts body tissue may engage with a reusable portion 34 of the device that is positioned within the sheath and isolated from body tissue. FIG. 7A illustrates one exemplary embodiment of sheath 1100 having a disposable portion 32 preloaded to the distal end 12 thereof. The disposable portion 32 may include an end effector 38 at its distal end, and may mate with a reusable portion 34 that extends through the second lumen 22 of the sheath 1100. The disposable and reusable portions 32, 34 together form the device 30. The reusable portion 34 may join with the disposable portion 32 at an interface 36 within the sheath 1100. The interface 36 may include features configured to transmit relative motion and/or signals between the coupled reusable and disposable portions 34, 32 to enable the end effector 38 (at the distal end of the disposable portion 32) to be controlled by an actuation device at the proximal end of the reusable portion 34. The interface 36 may also be configured to prevent biological tissue from contacting the reusable portion 34 within the sheath 1100. In some embodiments, the interface 36 may also be configured to prevent or minimize biological fluids from entering the sealed portion of the second lumen 22 that contains the reusable portion 34 of device 30. In some embodiments, the sheath 1100 may be thickened at selected regions to better seal the second lumen 22. In some embodiments, the disposable portion 32 may pierce through a seal at distal end of the second lumen 22 to interface with a distal end of the reusable portion 34 extending through the second lumen 22. The seal may remain functional to prevent or minimize the flow of biological fluids from entering the second lumen 22.

Any type of interface that enables the disposable and reusable portions 32, 34 to be coupled together while isolating the reusable portion 34 from body tissue may be used as interface 36. Interface 36 may be an integral part of one of the portions (such as, for example, an integral part of the disposable portion 32 as illustrated in FIG. 7A), or may be a part of the sheath 1100. FIG. 7B illustrates an exemplary interface 136 that is part of the sheath. In such an embodiment, the disposable and reusable portions 32, 34 of the device 30 may be separately coupled to the interface 136. In an embodiment using a device 30 with disposable and reusable portions 32, 34, the sheath 1100 along with the disposable portion 32 may be disposed after use while the reusable portion 34 may be reused. In general, any portion of a device can be the reusable portion 34. For instance, in some embodiments, only a portion of the end effector 38 may be disposable while the remaining portions of the device 30 may be reusable. In other embodiments, only a portion of the actuation device may be reusable while the remaining portions of the device 30 may be disposable.

In some exemplary medical procedures using a device 30 with disposable and reusable portions 32, 34, the two portions may be first coupled together before the sheath 1100 is inserted into the body. In other embodiments, only one of the portions (such as for example, the disposable portion 32) may be coupled to the sheath 1100 before the sheath 1100 is inserted into to the body. The remaining portion (such as, for example, the reusable portion 34) may be coupled after the sheath 1100 is inserted into to the body.

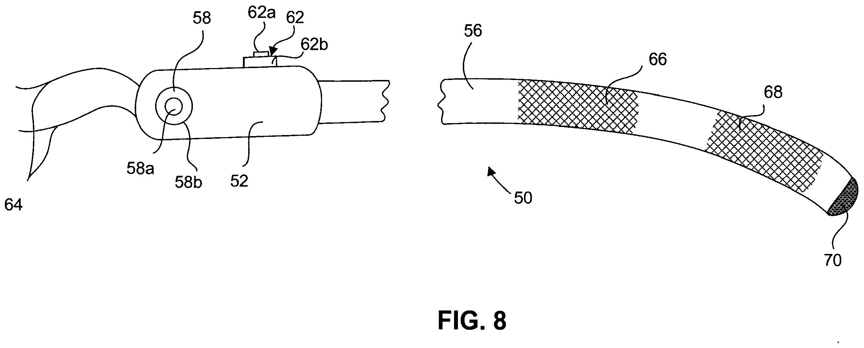

FIG. 8 illustrates a reusable device 50 that may be directed into the body though a sealed first lumen 20 of a disclosed sheath. Any device (such as, for example, a light wand, an imaging device, an endoscope, etc.) may serve as the reusable device 50. The reusable device 50 may include a flexible elongate section 56 extending from a handle 52. The handle 52 may include controls 58a, 58b, 62a, 62b that may activate different features of the reusable device 50. The elongate section 56 may include one or more bendable sections, such as, for example, a first bendable section 66 and a second bendable section 68, positioned along its length. These bendable sections 66, 68 may be configured to bend or deflect in response to the actuation of the controls 58a, 58b, 62a, 62b and have a curved disposition. In some embodiments, some of these controls (such as, for example, control 58a, 58b) may actuate the first bendable section 66 and the other controls (such as, for example, 62a, 62b) may actuate the second bendable section 68. Other embodiments of reusable devices may be controlled in a different manner. For example, in some embodiments, control 58a may be actuated to deflect the first bendable section 66 along a horizontal axis (that is, left/right) and control 58b may be actuated to deflect the second bendable section 68 along a horizontal axis. Similarly, controls 62a and 62b may actuate the first and second bendable sections 66, 68, respectively, along a vertical axis (up/down). These controls may actuate the bendable sections in any manner. In some embodiments, these controls may, mechanically, thermally, or electrically actuate the bendable sections to deflect in a desired direction. In other embodiments, the bendable sections may incorporate features (such as a fluid cavity, etc.) or devices (such as, actuators) that assist in the deflection of the elongate section 56 in a desired direction. Cables 64 (or conduits) may direct signals (and/or fluids) to the reusable device 50 to assist in the deflection of the bendable sections. In some embodiments, dynamic materials (such as, for example, shape memory materials, electroactive materials, etc.) may be used to actuate one or more bendable sections of the reusable device 50.

The distal-most end of elongate section 56 may include an optical element 70 that may receive and/or transmit light. Any device (such as, a light source, imaging device, etc.) that may emit light and/or may operate using received light may serve as the optical element 70. For example, optical element 70 may include a camera, imaging sensor (such as, for example, a complimentary metal-oxide semiconductor or a CMOS sensor, a CCD device, etc.), or another image receiving device (such as, for example, a fiber optic imaging device). Optical element 70 may transmit an image signal to a monitor or other display device positioned outside the body and viewable by the user. The image signals may correspond to still pictures and/or transient images that display time varying images of the work site within the body. In some embodiments, optical element 70 may transmit the image signals wirelessly, while in other embodiments, wires or cables embedded in the reusable device 50 may be used to transit these image signals. It is also contemplated that, in some embodiments, control signals may also be transmitted to the optical element 70 from outside the body, wirelessly or through the cables embedded in the reusable device 50. In addition to illumination devices 28 at the distal end 12 of a sheath, optical element 70 may also include an illumination device that is configured to illuminate the work site. The illumination device may include, among others, bulbs, LEDs, fiber optic cables and light guides. In some embodiments, the second bendable section 68 may be positioned proximate the optical element 70 and the first bendable section 66 may be positioned proximal to the second bendable section 68.

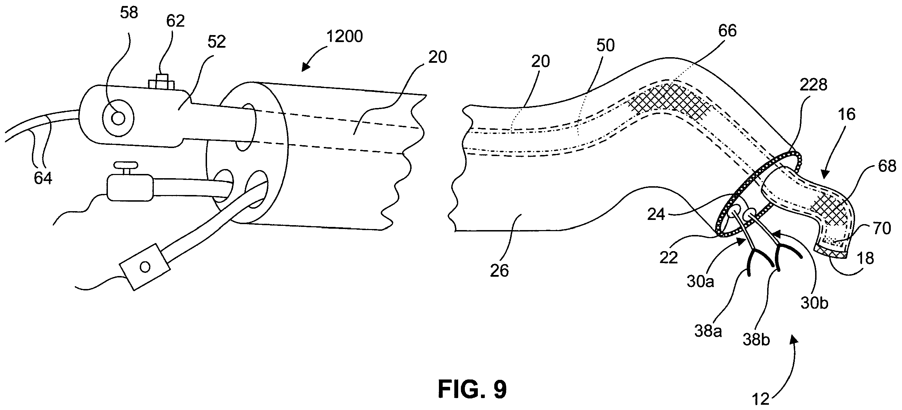

FIG. 9 illustrates an embodiment of sheath 1200 with a reusable device 50 extending through a first lumen 20 and two disposable devices 30a, 30b extending through the second and third lumens 22, 24, respectively. The reusable device 50 may be extended through the first lumen 20 to position the optical element 70 proximate the viewing lens 18. When the reusable device 50 is thus positioned, in some embodiments, the second bendable section 68 of the reusable device 50 may be positioned in the neck region 16 of the sheath 1200. In this configuration, actuating the second bendable section 68 to deflect this region of the reusable device 50 may be used to position the viewing lens 18 and the optical element 70 in any manner suited to view the work site (such as, for example, in an orientation suited to obtain an elevated view of the work site). Actuating the second bendable section 68 may also be used to guide or steer the sheath 1200 and the devices 30a, 30b extending through the second and third lumens 22, 24 to the work site. Actuating the first bendable section 66 may also be used to guide and position the distal end 12 of the sheath 1200 in a desirable manner within the body. Used together, the first and second bendable sections 66, 68 may be used to position the sheath 1200, and the devices therein, in a desirable manner within the body. For example, the first bendable section 66 may be actuated to raise the distal end 12 of sheath 1200 off the patients anatomy and the second bendable section 68 may be actuated to steer and direct the devices to a desired location and position the optics in a manner most suited to visualize the work site. The ring light 228 at the distal end 12 of sheath 1200 may be illuminated as desired to provide suitable illumination to the work site. Thus, a sheath of the current disclosure may be used to attach an optics lumen to a standard flexible disposable device. The sheath may also be used to provide steering capability to a standard non-articulating device by the use of a steerable device (such as, for example, an endoscope) in the sheath.

Sheath 10 (see FIG. 1) may be affixed to a device (reusable device 50 or disposable device 30) in any manner. In some embodiments, a device may be slid into (or squeezed into) a lumen of the sheath 10, while in other embodiments, the sheath 10 may be slid over the device. It is also contemplated that the sheath may be rolled over a device (similar to a condom), wrapped, zip-locked, hook and looped (such as, by using a Velcro® like attachment mechanism), adhered or expanded on the device. Expanding the sheath 10 on a device may include reversibly expanding the sheath 10 (by, for example, inflating with air or another fluid, by using temperature or a chemical change to increase or decrease the size of a lumen, etc.) to insert a device through the lumen and then contracting the lumen. In some embodiments, the device may be attached to the sheath 10 using features such as mechanical ties, "O" rings, clamps, etc.

The sheath may attached to a device in any order without limitation. For example, in some exemplary medical procedures, all the devices (reusable device 50 and disposable device 30a, 30b, etc.) may be first inserted into the sheath 10 (or otherwise attached to the sheath) before the sheath 10 (along with the inserted devices) is inserted into the body of the patient. In other embodiments, only some of the devices may be inserted into the sheath 10 before the sheath 10 is inserted into the body. The other devices may be inserted into the sheath 10 after the sheath 10 is within the body. For example, a reusable device 50 may first be inserted into the sheath 10 before the sheath 10 is inserted into the body of the patient. Disposable devices 30a, 30b, etc. may be inserted into the second and third lumens 22, 24 of the sheath 10 after the sheath is in the body. It is also contemplated that, in some exemplary medical procedures, one or more devices may be first inserted into the body before the sheath is slid over these devices. Other devices may then be inserted through the lumens of the sheath. It is also contemplated that, in some embodiments, the sheath 10 may be first inserted into the body before the devices are inserted into their respective lumens. In some embodiments, a seal may be formed around portions of the disposable devices to prevent or minimize the entry of biological fluids into the sheath 10.

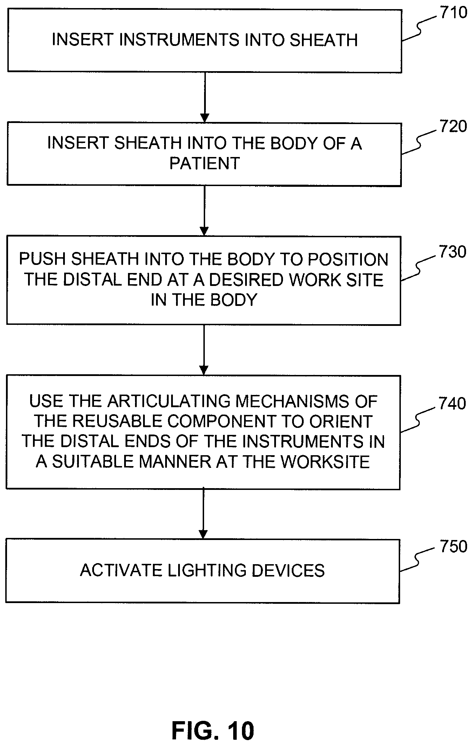

FIG. 10 is a flow chart that illustrates an exemplary method of using a sheath of the current disclosure. As illustrated in FIG. 10, a reusable device 50 (such as an endoscope) may be inserted into a closed lumen 20 and one or more disposable devices (30a, 30b, etc.) may be inserted into one or more open lumens 22, 24 of the sheath 10 (step 710). Although these devices are described as being inserted into the sheath, as discussed above, these devices may be attached in any manner to sheath 10. The sheath 10, along with the devices, therein may then be inserted into the body of a patient (step 720). The sheath 10 may be inserted into the body through a natural anatomic opening or through an incision on the body of the patient. The sheath 10 may be pushed into the body to a work site within the body (step 730). The articulating mechanisms (such as, the first and/or second bendable sections 66, 68) of the reusable device 50 may be activated to steer and guide the sheath through the body and position the distal end 12 of the sheath 10, the viewing lens 18, and the end effectors 38a, 38b in a desired configuration at the work site (step 740). In some embodiments, the neck region 16 of the first lumen 20 may be manipulated to position the viewing lens 18 over the work site to get an elevated view of the work site. The lighting devices at the distal end 12 of sheath 10 (such as, lighting devices 28a, 28b, and 28c of FIG. 1) may then be activated (step 750).

Although FIG. 10 shows the lighting devices as being activated after positioning the distal end of sheath 10 in the work site (that is, after step 740), this is only exemplary. In some embodiments, it may be desirable to activate one or more of the lighting devices before this step (step 740) to assist in directing the sheath 10 into the body. In these embodiments, some or all of the lighting devices may be activated during or before the sheath 10 is pushed into the body (step 730). It is also contemplated that some or all of the lighting devices may be activated before the sheath 10 is inserted into the body (step 720). In general, the lighting devices may be activated at any time (that is, before any of the steps described above) to illuminate the work site in the desired manner. In some embodiments, some of the lighting devices may be activated to assist in directing the sheath 10 into the body and/or assisting in suitably positioning the distal end 12 in the work site (step 740). After the distal end 12 is appropriately positioned in the work site, other lighting devices may be activated to assist in the medical procedure. In some embodiments, the lighting devices may be controlled based on feedback received from the reusable device 50. That is, based on the observed image of the work site, the illumination pattern of the lighting devices 28a, 28b, and 28c may be controlled to improve the illumination of the work site.

Although in the description above, the devices are described as being inserted into the sheath 10 before the sheath 10 is inserted into the body, as described previously, this is only exemplary and in other embodiments, sheath 10 (alone or with some devices inserted therein) may be inserted into the body before the devices are inserted into the sheath 10.

The embodiments described herein are exemplary only, and it will be apparent to those skilled in the art that various modifications and variations can be made in the disclosed systems and processes without departing from the scope of the invention. Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A sheath (10) for a medical device, comprising:
an elongate body (26) extending from a proximal end (14) to a distal face (12a);
a first hollow lumen (20) and a second hollow lumen (22) extending from a first end proximate the proximal end (14) to a second end proximate the distal face (12a);
at least one lighting device (28; 128; 228; 328; 428; 528) positioned in the sheath (10) proximate the distal face (12a);
a transparent window (18) positioned at the second end of the first lumen (20) to seal the first lumen (20) at the second end, the transparent window (18) being configured to transmit light therethrough; and
a second transparent window positioned at the second end of the second lumen (22) to seal the second lumen (22) at the second end, the transparent window being configured to transmit light therethrough.

2. The sheath of claim 1, wherein the at least one lighting device includes a plurality of lighting devices (28; 328; 428; 528) spaced apart from each other.

3. The sheath of claim 2, wherein the plurality of lighting devices (28; 228) are symmetrically positioned with respect to a longitudinal axis of the sheath (10).

4. The sheath of claim 1, wherein the at least one lighting device includes a plurality of lighting devices (228) positioned as a ring along a periphery of the sheath (10).

5. The sheath of claim 1, wherein the transparent window (18) at the second end of the first lumen (20) and the second transparent window at the second end of the second lumen (22) are part of a same transparent window.

6. The sheath of claim 1, wherein the sheath (10) includes a first neck region (16) that extends from the distal face (12a) to the second end of the first lumen (20), at least a portion of the first lumen being (20) positioned in the first neck region (16).

7. The sheath of claim 6, wherein the sheath (10) further includes a second neck region that extends from the distal face (12a) to the second end of the second lumen (22), at least a portion of the second lumen (22) being positioned in the neck region.

8. The sheath of claim 1, wherein the at least one lighting device (428) is positioned proximate a periphery of the second end of the first lumen (20).

9. The sheath of claim 1, further including a third lumen (24) extending from the proximal end (14) to the distal face (12a), the third lumen (24) being open at the distal face (12a).

## Patentansprüche

1. Hülle (10) für ein Medizinprodukt, die aufweist:
einen länglichen Körper (26), der sich von einem proximalen Ende (14) zu einer distalen Fläche (12a) erstreckt;
ein erstes hohles Lumen (20) und ein zweites hohles Lumen (22), die sich von einem ersten Ende nahe dem proximalen Ende (14) zu einem zweiten Ende nahe der distalen Fläche (12a) erstrecken;
mindestens ein Beleuchtungselement (28; 128; 228; 328; 428; 528), das in der Hülle (10) nahe der distalen Fläche (12a) positioniert ist;
ein transparentes Fenster (18), das am zweiten Ende des ersten Lumens (20) positioniert ist, um das erste Lumen (20) am zweiten Ende abzudichten, wobei das transparente Fenster (18) so konfiguriert ist, dass es Licht durchlässt; und
ein zweites transparentes Fenster, das am zweiten Ende des zweiten Lumens (22) positioniert ist, um das zweite Lumen (22) am zweiten Ende abzudichten, wobei das transparente Fenster so konfiguriert ist, dass es Licht durchlässt.

2. Hülle nach Anspruch 1, wobei das mindestens eine Beleuchtungselement mehrere Beleuchtungselemente (28; 328; 428; 528) aufweist, die voneinander beabstandet sind.

3. Hülle nach Anspruch 2, wobei die mehreren Beleuchtungselemente (28; 228) im Hinblick auf eine Längsachse der Hülle (10) symmetrisch positioniert sind.

4. Hülle nach Anspruch 1, wobei das mindestens eine Beleuchtungselement mehrere Beleuchtungselemente (228) aufweist, die als Ring entlang eines Umfangs der Hülle (10) positioniert sind.

5. Hülle nach Anspruch 1, wobei das transparente Fenster (18) am zweiten Ende des ersten Lumens (20) und das zweite transparente Fenster am zweiten Ende des zweiten Lumens (22) Teile desselben transparenten Fensters sind.

6. Hülle nach Anspruch 1, wobei die Hülle (10) einen ersten Halsbereich (16) aufweist, der sich von der distalen Fläche (12a) zum zweiten Ende des ersten Lumens (20) erstreckt, wobei mindestens ein Abschnitt des ersten Lumens (20) im ersten Halsbereich (16) positioniert ist.

7. Hülle nach Anspruch 6, wobei die Hülle (10) ferner einen zweiten Halsbereich aufweist, der sich von der distalen Fläche (12a) zum zweiten Ende des zweiten Lumens (22) erstreckt, wobei mindestens ein Abschnitt des zweiten Lumens (22) im Halsbereich positioniert ist.

8. Hülle nach Anspruch 1, wobei das mindestens eine Beleuchtungselement (428) nahe einem Umfang des zweiten Endes des ersten Lumens (20) positioniert ist.

9. Hülle nach Anspruch 1, ferner mit einem dritten Lumen (24), das sich vom proximalen Ende (14) zur distalen Fläche (12a) erstreckt, wobei das dritte Lumen (24) an der distalen Fläche (12a) offen ist.

## Revendications

1. Gaine (10) pour un dispositif médical, comprenant :
un corps allongé (26) s'étendant à partir d'une extrémité proximale (14) jusqu'à une face distale (12a) ;
une première lumière creuse (20) et une deuxième lumière creuse (22) s'étendant à partir d'une première extrémité à proximité de l'extrémité proximale (14) jusqu'à une seconde extrémité à proximité de la face distale (12a) ;
au moins un dispositif d'éclairage (28 ; 128 ; 228 ; 328 ; 428 ; 528) positionné dans la gaine (10) à proximité de la face distale (12a) ;
une fenêtre transparente (18) positionnée au niveau de la seconde extrémité de la première lumière (20) pour sceller la première lumière (20) au niveau de la seconde extrémité, la fenêtre transparente (18) étant configurée pour transmettre la lumière à travers cette dernière ; et
une seconde fenêtre transparente positionnée au niveau de la seconde extrémité de la deuxième lumière (22) pour sceller la deuxième lumière (22) au niveau de la seconde extrémité, la fenêtre transparente étant configurée pour transmettre la lumière à travers cette dernière.

2. Gaine selon la revendication 1, dans laquelle le au moins un dispositif d'éclairage comprend une pluralité de dispositifs d'éclairage (28 ; 328 ; 428 ; 528) espacés les uns des autres.

3. Gaine selon la revendication 2, dans laquelle la pluralité de dispositifs d'éclairage (28 ; 228) sont symétriquement positionnés par rapport à un axe longitudinal de la gaine (10).

4. Gaine selon la revendication 1, dans laquelle le au moins un dispositif d'éclairage comprend une pluralité de dispositifs d'éclairage (228) positionnés comme une bague le long d'une périphérie de la gaine (10).

5. Gaine selon la revendication 1, dans laquelle la fenêtre transparente (18) au niveau de la seconde extrémité de la première lumière (20) et la seconde fenêtre transparente au niveau de la seconde extrémité de la deuxième lumière (22) font partie d'une même fenêtre transparente.

6. Gaine selon la revendication 1, dans laquelle la gaine (10) comprend une première région de col (16) qui s'étend à partir de la face distale (12a) jusqu'à la seconde extrémité de la première lumière (20), au moins une partie de la première lumière (20) étant positionnée dans la première région de col (16).

7. Gaine selon la revendication 6, dans laquelle la gaine (10) comprend en outre une seconde région de col qui s'étend à partir de la face distale (12a) jusqu'à la seconde extrémité de la deuxième lumière (22), au moins une partie de la deuxième lumière (22) étant positionnée dans la région de col.

8. Gaine selon la revendication 1, dans laquelle le au moins un dispositif d'éclairage (428) est positionné à proximité d'une périphérie de la seconde extrémité de la première lumière (20).

9. Gaine selon la revendication 1, comprenant en outre une troisième lumière (24) s'étendant partir de l'extrémité proximale (14) jusqu'à la face distale (12a), la troisième lumière (24) étant ouverte au niveau de la face distale (12a).
